# EUROPEAN PATENT SPECIFICATION

(11) **EP 1 729 777 B1**
(45) Date of publication and mention of the grant of the patent: **04.03.2009**
(21) Application number: 05740221.6
(22) Date of filing: 19.04.2005
(51) Int. Cl.: A61K 31/55, C07D 471/22

(54) **PHARMACEUTICAL COMPOSITION COMPRISING A SALT OF MIRTAZAPINE**
EIN SALZ VON MIRTAZAPIN ENTHALTENDE PHARMAZEUTISCHE ZUSAMMENSETZUNG
COMPOSITION PHARMACEUTIQUE COMPRENANT UN SEL DE MIRTAZAPINE

(30) Priority: 21.04.2004 EP 04101664
(43) Date of publication of application: 13.12.2006
(73) Proprietor: N.V. Organon, 5349 AB Oss (NL)
(72) Inventor: MOOLENAAR, Sytske, Hyke, NL-5340 BH OSS (NL); KEMPERMAN, Gerardus, Johannes, NL-5340 BH Oss (NL); VAN DER VOORT MAARSCHALK, Kees, NL-5340 BH OSS (NL)
(74) Representative: Broekkamp, Chris L. E.
(86) International application number: PCT/EP2005/051714
(87) International publication number: WO 2005/102352

(56) References cited:
- EP-A- 0 813 873
- WO-A-01/00196
- WO-A-01/19371
- US-A- 4 062 848
- US-A- 6 114 324
- FINK M ET AL: "PHARMACO-EEG STUDY OF 6-AZAMIANSERIN (ORG 3770): DISSOCIATION OF EEG AND PHARMACOLOGIC PREDICTORS OF ANTIDEPRESSANT ACTIVITY" PSYCHOPHARMACOLOGY, SPRINGER VERLAG, BERLIN, DE, vol. 78, no. 1, 1982, pages 44-48, XP009032494 ISSN: 0033-3158 cited in the application
- GOULD P L: "SALT SELECTION FOR BASIC DRUGS" INTERNATIONAL JOURNAL OF PHARMACEUTICS, AMSTERDAM, NL, vol. 33, no. 1/3, 1986, pages 201-217, XP002074725 ISSN: 0378-5173 cited in the application

## Description

The invention relates to pharmaceutical formulations comprising a pure enantiomer of mirtazapine.

Mirtazapine is a widely used drug with several therapeutic uses. The form of the drug which is available in pharmaceutical compositions for prescribing to patients is the base of the compound as racemic mixture. In view of new uses of the drug and the different pharmacological properties of the enantiomers it is needed to make the separate single Sand R-enantiomers available for pharmaceutical compositions. Pharmaceutical compositions for oral use of enantiomers were implied to be available according to the publication of Fink and Irwin (Psychopharmacology, Vol 78, pp. 44-48, 1982) who describe the administration of the S-enantiomer and the R-enantiomer of mirtazapine to human volunteers for research purposes. The compounds were given in the form of the free base of an S- or R- enantiomer of mirtazapine.

It was found that such formulations suffer from problems caused by sublimation of the mirtazapine. It was found that the pure bases of S- and R-mirtazapine are slowly sublimating compounds at ambient temperature and that some, but not all salts of S- and R-mirtazapine do not have this disadvantage. Thus, the usefulness of such a pharmaceutical composition comprising an enantiomer of mirtazapine in solid form can be improved, according to this invention, by selecting a pharmaceutically suitable non-sublimating and solid salt of an enantiomer of mirtazapine for use as the form of mirtazapine in the composition. The invention also provides for a method to prevent sublimation of mirtazapine from a pharmaceutical formulation comprising a pure enantiomer of mirtazapine in a solid form by making the formulation whereby the solid form is a pharmaceutically suitable non-sublimating salt of S- or R-mirtazapine.

Other desirable properties for a pharmaceutical ingredient, such as ease in preparation or purification or chemical or physical stability in capsules and/or tablets can also be obtained by use of a salt according to this invention. Improved physical stability can be due to reduced migration of compound out of the formulation and improved chemical stability can be due to reduced degradation of mirtazapine. Non-sublimating and solid salts of S-mirtazapine as well as R-mirtazapine are found to be a.o. the maleic acid, the hydrochloric acid, the hydrobromic acid, the fumaric acid and the methanesulfonic acid salts of mirtazapine. The maleic acid salt is particularly advantageous, because it has a high melting point, readily forms crystals, for which there form no other polymorphs and it is not hygroscopic. Also, methanesulfonic acid salt is a very useful salt for an enantiomer of mirtazapine, in view of non-sublimation and non-hygroscopicity. A trifluoroacetic acid salt of S- or R-mirtazapine is an example of a salt which showed sublimation. Moreover, the latter salt is not a pharmaceutically suitable salt.

The property of sublimation can be observed and quantified with known methods to measure sublimation. For example, sublimation can be measured in an apparatus with a chamber in which the test compound is placed in its solid state and maintained in that state by controlled temperature. The gas phase in the chamber, optionally under low pressure, can be analyzed for content of test compound. It is also possible to continuously clear the test compound in the gas phase from the chamber either by a continuous renewal stream of a gas or by creating a sink for the test compound out of the gas phase, for example by a cold surface. The amount of material collected from sublimation or escaped from the sample by sublimation can be analyzed. The degree of sublimation is expressed as the fraction (as percentage) of the initial sample size.

The term 'non-sublimating salt' is defined to be a salt of S- or R-mirtazapine, from which less than 1 % of the mirtazapine is sublimating from the sample, calculated on the basis of the amount of the base, when a sample of approximately 10 mg (for example an amount between 8-12 mg) is placed for the duration of 72 hours under standard conditions of 150 mBar pressure and 60°C temperature.

Pharmaceutically suitable acids approved for use to provide for the anion in a salt of a medicinally active compound are hydrochloric acid, hydrobromic acid, sulfuric acid, maleic acid, fumaric acid, methylsulfonic acid, acetic acid, and other acids mentioned in the article of Philip L. Gould. (international Journal of Pharmaceutics, Vol. 33, (1986), pp. 201-207. This publication provides for the limiting and defined list of acids, which can be tested according to prescribed procedures in this description to obtain a salt according to the invention.

The term 'solid' in this description means that the amorphic or crystalline compound remains in a solid state at room temperature.

The term mirtazapine refers to the compound 1,2,3,4,10,14b-hexahydro-2-methylpyrazino[2,1-a]pyrido[2,3-c][2]benzazepine as active ingredient for a pharmaceutical formulation. The term is used here to refer to the free base as separate compound or to the base component in a mirtazapine salt.

Reference to a formulation comprising an enantiomer of mirtazapine refers to a formulation in which an enantiomerically purified form of mirtazapine was used in the preparation, contrary to a formulation for which the racemic form of mirtazapine was used. Purification in this paragraph is meant to implicate one or more steps in the preparation of mirtazapine, which are aimed at obtaining some degree of separation of the two enantiomers. Preferably a pure enantiomer of 90%, or preferably better up to 95%, 98%, 99%, 99.5% or 99.8% purity over the other enantiomer is used

Mirtazapine, 1,2,3,4,10,14b-hexahydro-2-methyl-pyrazino[2,1-a]pyrido[2,3-c][2]benzazepine, can be prepared by known methods. Synthesis of racemic mirtazapine is described, for example, in US4062848 wherein a four stage synthetic scheme is disclosed starting from a 2-substituted nicotinitrile. Further modifications to various stages of this route have subsequently been described in WO 00/62782, WO 01/23345 and US 6,376,668.

The preparation of enantiomerically pure mirtazapine has been addressed in US 4062848, WO 00/62782 and Selditz et. al., 1998 (J. Chromatography, 1998, vol 803, pp 169-177). By the method disclosed in US 4062848, enantiomerically pure mirtazapine is obtained by fractional crystallisation of the diastereoisomeric salts formed by reaction of racemic mirtazapine with enantiomerically pure dibenzoyltartaric acid in ethanol followed by regeneration of the free base by treatment with aqueous ammonia. Other methods of forming pure mirtazapine by recrystallisation of crude mirtazapine are disclosed in WO 00/62782. Selditz et. al. describe a chromatographic method to separate the enantiomers.

Pharmaceutical compositions are made with an active ingredient, which is a salt of S- or R-mirtazapine in this context, to which carriers and other excipients are added.
The characteristics of the salts according to the invention make them most suitable for manufacture and use in various pharmaceutical formulations for dosaged administration to a subject. Such forms are adapted to use for particular routes of administration, such as oral, rectal or transdermal.

For making dosage forms, such as pills, tablets, suppositories, (micro-)capsules, powders, emulsions, creams, ointments, implants, a patch, a gel, or any other preparation for sustained release, sprays, injection preparations in the form of a suspension, suitable auxiliaries such as fillers, binders, lubricants, dispersants, emulsifiers, stabilisers, surfactants, penetration enhancers, anti-oxidants, colorants and preservatives can be used e.g. as described in the standard reference, Gennaro et al., Remington; The Science and Practice of Pharmacy; 20th ed., Publisher: Lippincott Williams & Wilkins; Baltimore; USA in Part 5) and the Handbook of Pharmaceutical Excipients (3nd edition edited by Arthur H. Kibbe; Published by the American Pharmaceutical Association, Washington D.C. and The Pharmaceutical Press, London in 2000). In general any pharmaceutically acceptable auxiliary which does not interfere with the function of the active compound is suitable and can be used. The amount of S- or R-mirtazapine salt in the dosage form can be adapted to the particular circumstances. Generally, a dosage unit will contain between 0.05 and 90 mg of S- or R-mirtazapine salt, expressed on the basis of the amount of base.

Suitable fillers or carriers with which the compositions can be administered include agar, alcohol, fats, lactose, starch, cellulose derivatives, polysaccharides, polyvinylpyrrolidone, silica, and sterile saline, or mixtures thereof, used in suitable amounts.

Binders are agents used to impart cohesive properties to a pharmaceutical composition resulting in minimal loss from the pharmaceutical composition during production and handling. Binders are for example cellulose, starches and polyvinylpyrrolidone.

A suitable lubricant with which the active agent of the invention can be administered is, for example, magnesium stearate.

Surfactants are agents facilitating the contact and migration of compounds in different physical environments such as hydrophilic and hydrophobic environments. Many surfactants are known in the art of making pharmaceutical compositions as for example described in chapter 21 of Gennaro et al, Remington; The Science and Practice of Pharmacy; 20th ed., Publisher. Lippincott Williams & Wilkins; Baltimore; USA). Surfactants that can be used during the process of preparing the pharmaceutical formulation are, for example and polyethyene glycol (PEG).

The salts according to the invention can be made with methods well-known in the art. The base is dissolved in a suitable solvent, such as methanol, ethanol, ethylacetate or acetone and acid is added either purely or dissolved in, for example ethanol, ethylacetate or acetone. The salt can be collected from the solvens by precipitation or crystallisation, which is, if needed, provoked by cooling the solution or evaporating the solventia.

**Figure**: Schematic presentation of the sublimation test equipment. A sample is placed on the bottom of a vessel, which is closed at the top by a vessel-shaped stopper into which cooling liquid (CL) is circulating and which vessel has an outlet connected to a vacuum pump (Vac). The vessel is placed in a closed chamber under constant temperature control (TC). A sublimate (Subl) can accumulate against the surface of the stopper within the vessel.

### Examples

In the examples S-mirtazapine is used. In view of the symmetry these can be directly copied to apply to R-mirtazapine as well, except for the example 8, in which case (-)-O,O-dibenzoyl-L-tartaric acid must be used for R-mirtazapine.

### 1. Crystallization of S-mirtazapine hydrochloric acid salt

To a solution of 3.01 g of S-mirtazapine in 5 ml of methanol was added at room temperature a solution of 939 µl of hydrochloric acid in 20 ml of ethyl acetate. Part of the solvent was evaporated and an oil was formed in the solution. Then the solution was cooled to 0 °C. A seed crystal was added whereupon crystallisation started. The white crystals were collected by filtration and were dried in a vacuum oven at 40°C. This gave 1.96 g of white crystals of S-mirtazapine hydrochloric acid salt (57%).

Endothermic peak (DSC): 275°C; XRPD and ss-NMR: crystalline material of one polymorhic form, no amorphous material. The compound starts to sublime above 170°C. Dynamic vapor sorption measurement demonstrated that the salt is very hygroscopic.

### 2. Crystallization of S-mirtazapine maleic acid salt

To a solution of 3.01 g of S-mirtazapine in 10 ml of ethanol was added at room temperature a solution of 1.32 g of maleic acid in 10 ml of ethanol. After stirring for several minutes crystallization started. After stirring for several hours at room temperature the white crystals were collected by filtration and were dried in a vacuum oven at 40°C. This gave 3.98 g of white crystals of S-mirtazapine maleic acid salt (92%).

Endothermic peak (DSC): 206°C; XRPD and ss-NMR: crystalline material; ratio mirtazapine/maleic acid: 1:1; one polymorphic form, no amorphous material. Dynamic vapor sorption measurement demonstrated that the salt is not hygroscopic.

### 3. Crystallization of S-mirtazapine fumaric acid salt

To a solution of 3.01 g of S-mirtazapine in 5 ml of methanol was added at room temperature 1.31 g of fumaric acid resulting in a quick precipitation. An additional of 5 ml of methanol and 20 ml of ethyl acetate were added to the suspension to redissolve the solid. Part of the solvent was evaporated to start the crystallization from a clear solution. After stirring for several hours, the white crystals were collected by filtration and were dried in a vacuum oven at 40°C. This gave 3.76 g of white crystals of S-mirtazapine fumaric acid salt (87%). Endothermic peak (DSC): 178°C; XRPD and ss-NMR: probably a mixture of three polymorphic forms and some amorphous material. The fumaric acid salt attracts water from environmental air to form a hydrate, which can loose its water content upon drying.

### 4. Crystallization of S-mirtazapine hydrobromic acid salt

To a solution of 3.01 g of S-mirtazapine in 5 ml of methanol was added at room temperature a solution of 1290 µl of hydrobromic acid in 20 ml of ethyl acetate. Part of the solvent was evaporated, which resulted in the formation of an oil. The mixture was cooled to 0 °C whereupon crystallization started. The white crystals were collected by filtration and were dried in a vacuum oven at 40°C. This gave 3.74 g white crystals of S-mirtazapine hydrobromide salt (95%). Endothermic peak (DSC): 253 °C; XRPD and ss-NMR: mainly one polymorphic form and some amorphous material. The HBr salt has a clear affinity for water and forms a monohydrate under ambient conditions. A water free drug substance sample attracts water when it comes in contact with environmental air, while it may loose water upon drying.

### 5. Crystallization of S-mirtazapine methanesulfonic acid salt

To a solution of 3.01 g of S-mirtazapine in 5 ml of methanol was added at room temperature a solution of 743 µl of methanesulfonic acid in 20 ml of ethyl acetate. After partly evaporation of the solvent, the crystallization started. The white crystals were collected by filtration and were dried in a vacuum oven at 40°C. This gave 2.09 g white crystals of S-mirtazapine methanesulfonic salt (51%). Endothermic peak (DSC): 208°C; XRPD and ss-NMR: crystalline material mainly one polymorph.

### 6. Crystallization of S-mirtazapine trifluoroacetic acid salt

To a solution of 0.50 g of S-mirtazapine in ethyl acetate was added a solution of 142 µl of trifluoroacetic acid in ethyl acetate. As crystallization did not start spontaneously the solvent was evaporated slowly. During evaporation of the solvent the salt started to crystallize. This yielded 0.65 g of S-mirtazapine trifluoroacetic acid salt. Endothermic peak: 185°C. In experiments according to example 10, it was found that this salt was not a non-sublimating salt according to the definition of a non-sublimating salt in this description.

### 7. Solidification of S-mirtazapine formic acid salt, S-mirtazapine acetic acid salt, S-mirtazapine propionic acid salt and S-mirtazapine phosphoric acid salt did not succeed.

### Example 8.

This is to demonstrate the first step in a manner of preparation of enantiomerically pure mirtazapine. The salt of this example is not approved for pharmaceutical use.

### Crystallization of S-mirtazapine (+)-O,O-Dibenzoyl-D-Tartaric acid salt

23.33 g mirtazapine (Org 3770) was dissolved in 94 ml of ethanol at a temperature of 52 °C. A filtered solution of 33.06 g (+)-O,O-dibenzoyl-D-Tartaric acid hydrate in 132 ml of ethanol (100%) was added to the warm solution. Then the reaction mixture was cooled down to room temperature. A seed crystal was added to the reaction mixture to initiate crystallization. After stirring for 19 hours, the crystals were collected by filtration. The yield of the wet crystals was 25.7 g and the e.e. was 88.04%. The crystals were suspended in 880 ml of ethanol and dissolved at reflux temperature. The reaction mixture was cooled down and crystallisation started. After 16 hours the crystals were collected by filtration. The yield of the wet crystals was 20.4 g and the e.e. was 98.9%. The remaining mother liquor can be used to obtain R-mirtazapine by combining with (-)-O,O-Dibenzoyl-L-Tartaric acid

### 9. Assay for S-mirtazapine and degradation products by HPLC

| | |
|---|---|
| Column | Hypersil ODS, 250 x 4.6 mm I.D., 5 µm or equivalent column |
| Column temperature | 40°C |
| Solution A | Methanol + acetonitrile + tetrahydrofuran, |
| | 36.2 + 42.5 + 21.3, V+V+V. |
| Mobile phase | Solution A + tetramethylammonium |
| | hydroxide pentahydrate solution 0.1 M |
| | (pH=7.4), |
| | 35 + 65, V+V |
| Flow rate | 1.5 mL/min |
| Detection | S-mirtazapine: UV 290 nm |
| | Degr prod A: UV 240 nm |
| | Degr prod B: UV 240 nm |
| | Degr prod C: UV 240 nm |
| | Degr prod D: UV 240 nm |
| Injection volume | 10 µL |
| Run time | 27 minutes |
| Approximate retention times * | |
| S-mirtazapine | 14.5 ≤ t_{R} ≤ 17.5 minutes |
| Degr prod A | 23.1 minutes |
| Degr prod B | 3.0 minutes |
| Degr prod C | 5.6 minutes |
| Degr prod D | 3.7 minutes |

| | |
|---|---|
| * If the retention time of the S-mirtazapine peak is not conform the system suitability criteria prior to analysis, the mobile phase should be adjusted by adding some solution A or tetramethylammonium hydroxide pentahydrate solution 0.1 M. | |

The detection limit of S-mirtazapine as free base or as active entity in its corresponding salt is below 0.02 mg.
Names:
Degr prod A: 2,3,4,4a-Tetrahydro-3-methylpyrazino[2,1-a]pyrido[2,3-c][2]benzazepine-9(1 H)-one
Degr prod B: 1,2,3,4,10,14b-Hexahydro-2-methylpyrazino[2,1-a]pyrido[2,3-c][2]benzazepine-2-oxide dihydrate
Degr prod C: 3,4,10,14b-Tetrahydro-2-methylpyrazino[2,1-a]pyrido[2,3-c][2]benzazepin-1 (2H)-one
Degr prod D: N-[2-(5,10-dihydro-10-oxo-11H-pyrido[2,3-c][2]benzazepin-11-yl)ethyl]-N-methyl-formamide

### 10. Sublimation tests

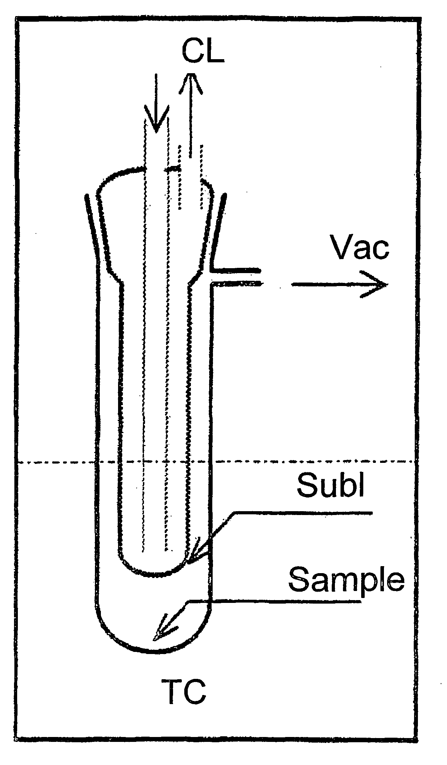

A sample (approximately 10 mg) was placed in a sample chamber of sublimation test equipment as illustrated in Figure 1. The temperature in the sample chamber is controllable. The sample holder has a reduced pressure, which was 150 mBar by default. The sample chamber also consists of a section with reduced temperature (the "cold finger"), where the temperature is approximately 5°C. The majority of material that sublimates in the test sample with high temperature will precipitate on the cold finger. The amount of material on the cold finger after a test period of 72 hrs has been quantified using HPLC analysis. The degree of sublimation is expressed as the fraction material on the cold finger (as percentage) of the initial sample size. Additionally, the sublimation of active compound from tablets "drug product" has been tested.

### Results

Table 1 lists the sublimation results of the S-mirtazapine, S-mirtazapine.HBr, S-mirtazapine.maleic acid, S-mirtazapine.fumaric acid and tablets containing some of these compounds under several test conditions.

**Table 1: Sublimation results**

| | | **Sublimate^{a}** |
|---|---|---|
| **Temperature: 40°C** | **Drug substance** | |
| **Pressure: 150 mbar** | S-mirtazapine Batch E | 0.75% |
| **Test period: 72 hrs** | S-mirtazapine Batch J | 0.88% |
| | | |

| **Temperature: 60°C** | **Drug substance** | |
|---|---|---|
| **Pressure: 150 mbar** | S-mirtazapine Batch E | 5.32%, 2.22% |
| **Test period: 72 hrs** | S-mirtazapine Batch J | 4.29% |
| | S-mirtazapine.HBr | <DL |
| | S-mirtazapine.maleic acid | <DL |
| | S-mirtazapine.fumaric acid | <DL |
| | S-mirtazapine.HCl | 0.2% |
| | | 0.2% |
| | S-mirtazapine.methanesulfonic acid | 0.1%, 0.0% |
| | | |

| | **Drug product (tablets)¹** | |
|---|---|---|
| | S-mirtazapine tablets² | 7.21% |
| | S-mirtazapine.HBr tablets | <DL |
| | S-mirtazapine.maieic acid tablets¹ | <DL |

| | | |
|---|---|---|
| <DL = Below level of detection ^{a} If two values are mentioned these are results of replication experiments. ¹ The composition of the tablets used for the sublimation test detailed in table 3. ² Formulation C as in table 3. | | |

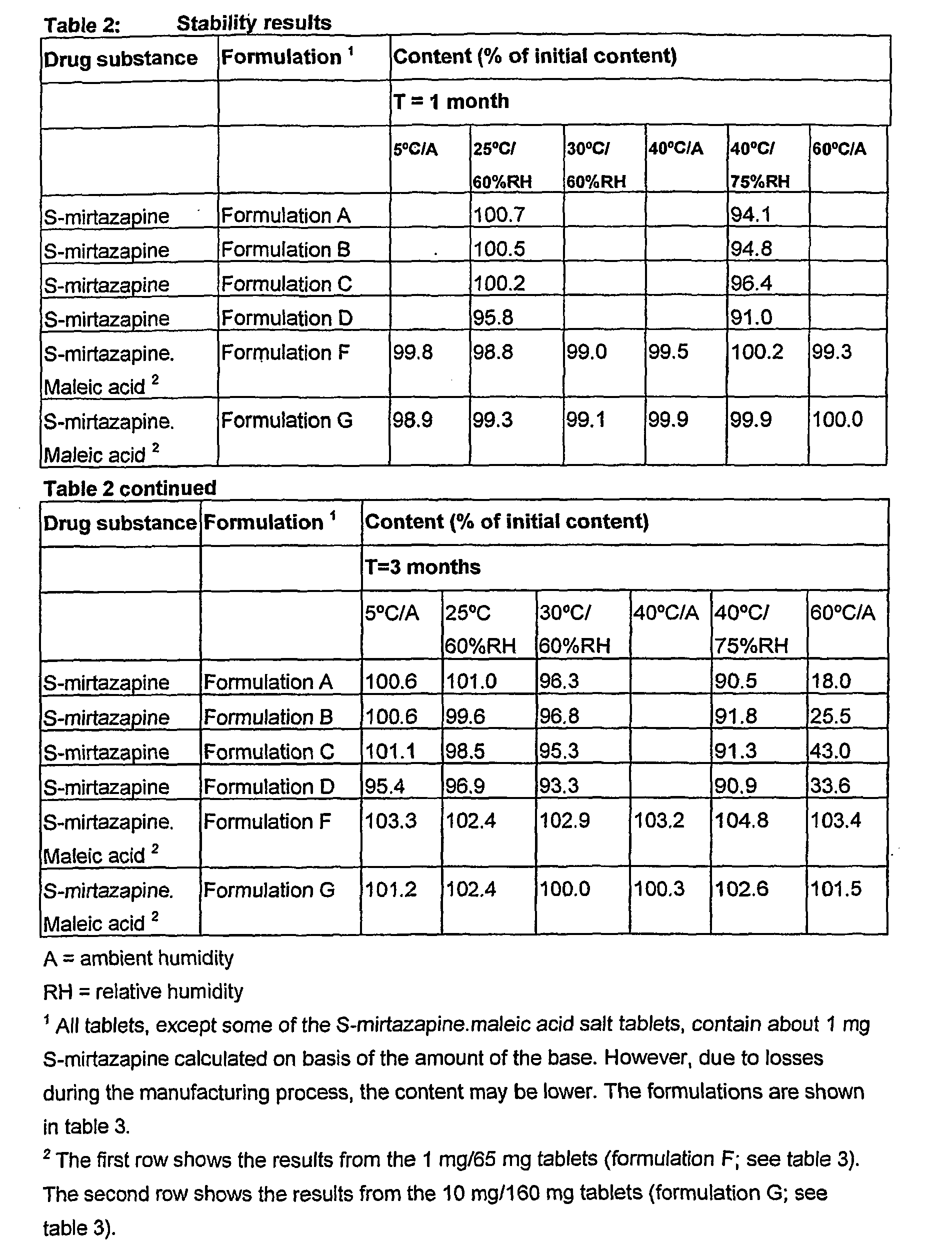

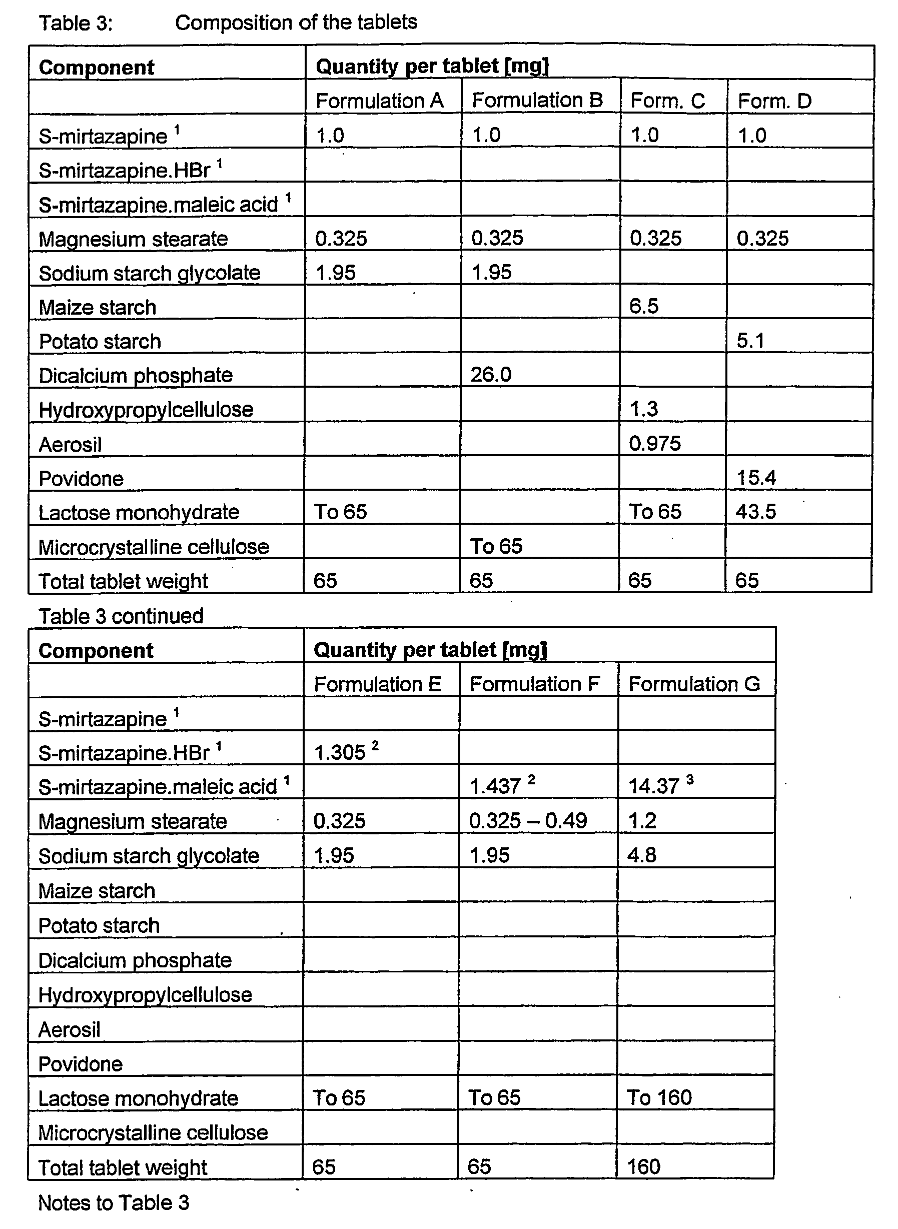

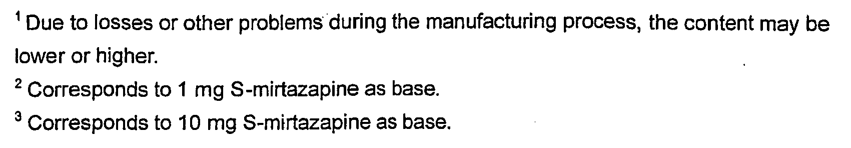

The results of the sublimation test show that the free base S-mirtazapine sublimates at the conditions used. The S-mirtazapine hydrobromic acid salt, S-mirtazapine maleic acid salt, and S-mirtazapine fumaric acid salt do not sublimate. This difference is also seen in tablets containing S-mirtazapine, S-mirtazapine.HBr, or S-mirtazapine.maleic acid. The stability of tablets containing S-mirtazapine and S-mirtazapine.maleic acid was also maesured (tables 2 and 4). It is clear that the S-mirtazapine content decreases in the tablets containing the free base S-mirtazapine. The decrease is probably caused by sublimation. In the tablets containing S-mirtazapine.maleic acid no decrease in the content was observed.

The chemical stability of the drug products was further analysed by determination of chemical degradation products. The assay values after storage are shown in table 4.

**Table 4: Stability results assay and degradation products**

| **Drug substance** | **Formulation ¹** | **T=3 months** | | | | | |
|---|---|---|---|---|---|---|---|
| | | **5°C/A** | **25°C 60%RH** | **30°C/ 60%RH** | **40°C/A** | **40°C/ 75%RH** | **60°C/A** |
| S-mirtazapine | Formulation A | 100.6 | 101.0 | 96.3 | | 90.5 | 18.0 |
| | | | | | | | |
| Degradation products | | | | | | | |
| Degr prod A | | <0,1% | <0.1% | <0.1% | | <0.1% | 0.53% |
| Degr prod B | | <0.1% | <0.1% | <0.1% | | 0.16% | 3.70% |
| Degr prod C | | <0.1% | <0.1% | <0.1% | | 0.14% | 1.06% |
| Degr prod D | | <0.1% | 0.11% | 0.14% | | 0.35% | 5.57% |
| S-mirtazapine.maleic acid | Formulation F | 103.3 | 102.4 | 102.9 | 103.2 | 104.8 | 103.4 |
| | | | | | | | |
| Degradation products | | n.d. | | | | | n.d. |
| Degr prod A | | | <0.1% | <0.1% | <0.1% | <0.1% | |
| Degr prod B | | | <0.1% | <0.1% | <0.1% | 0.18% | |
| Degr prod C | | | <0.1% | <0.1% | <0.1% | <0.1% | |
| Degr prod D | | | <0.1% | <0.1% | <0.1% | 0.11% | |

| | | | | | | | |
|---|---|---|---|---|---|---|---|
| Notes to Table 4 ¹ All tablets contain about 1 mg active entity (S-mirtazapine). However, due to losses during the manufacturing process, the active entity content may be lower. The formulations are shown in table 3. n.d. = not determined A = ambient humidity | | | | | | | |

Note that in the tablets containing S-mirtazapine.maleic acid no decrease in the content was observed. Degradation products found in tablets containing S-mirtazapine or S-mirtazapine.maleic acid are provided in Table 4. The formulation of the tablets is similar (table 3). In tablets containing S-mirtazapine.maleic acid stored for 3 months at 40°/75%RH the concentration of the degradation products was lower than in tablets containing the free base S-mirtazapine that were stored during the same period. Losses under conditions of 60°C at ambient temperature were not much more for the maleic acid salt, whereas for the formulation with the free base the degradation products already amount to about 11 %.

## Claims

1. A pharmaceutical formulation comprising an enantiomerically purified form of mirtazapine as a pharmaceutically suitable, non-sublimating and solid salt **characterised in that** the salt is of maleic acid or of methanesulfonic acid.

2. The pharmaceutical formulation according to claim 1, **characterised in that** the enantiomer of mirtazapine is the S-enantiomer:

3. A method to prevent sublimation of mirtazapine from a pharmaceutical formulation comprising an enantiomerically purified form of mirtazapine in a solid form by making the formulation by adding at least one pharmaceutically acceptable auxiliary to the solid form of the enantiomer of mirtazapine, **characterised in that** the enantiomerically purified form is a pharmaceutically suitable, non-sublimating and solid salt of S- or R-mirtazapine.

4. S-mirtazapine maleate, S-mirtazapine methanesulfonate, 5-mirtazapine fumarate hydrate or S-mirtazapine bromide monohydrate.

## Patentansprüche

1. Pharmazeutische Formulierung, umfassend eine enantiomerisch gereinigte Form von Mirtazapin als ein pharmazeutisch geeignetes, nicht sublimierendes und festes Salz, **dadurch gekennzeichnet, dass** das Salz von der Maleinsäure oder der Methan-Sulfon-Säure ist.

2. Pharmazeutische Formulierung nach Anspruch 1, **dadurch gekennzeichnet, dass** das Enantiomer von Mirtazapin das S-Enantiomer ist.

3. Verfahren zur Verhinderung der Sublimation von Mirtazapin aus einer pharmazeutischen Formulierung, umfassend eine enantiomerisch gereinigte Form von Mirtazapin in einer festen Form durch Herstellung der Formulierung durch Hinzufügen von mindestens einem pharmazeutisch verträglichen Hilfsstoff zur festen Form des Enantiomers von Mirtazapin, **dadurch gekennzeichnet, dass** die enantiomerisch gereinigte Form ein pharmazeutische geeignetes, nicht sublimierendes und festes Salz von S- oder R-Mirtazapin ist.

4. S-Mirtazapin-Maleat, S-Mirtazapin-Methansulfonat, S-Mirtazapin-Fumarathydrat, oder S-Mirtazapin-Bromidmonohydrat.

## Revendications

1. Formulation pharmaceutique comprenant une forme énantiomériquement purifiée de mirtazapine en tant que sel convenable pharmaceutiquement, non sublimant et solide, **caractérisée en ce que** le sel est un sel de l'acide maléique, ou un sel de l'acide méthanesulfonique.

2. La formulation pharmaceutique selon la revendication 1, **caractérisée en ce que** l'énantiomère de mirtazapine est le S-énantiomère.

3. Procédé pour prévenir la sublimation de la mirtazapine d'une formulation pharmaceutique comprenant une forme énantiomériquement purifiée de mirtazapine sous forme solide par préparation de la formulation en additionnant au moins un auxiliaire pharmaceutiquement acceptable à la forme solide de l'énantiomère de mirtazapine, **caractérisé en ce que** la forme énantiomériquement purifiée est un sel convenable pharmaceutiquement, non sublimant et solide de S- ou R-mirtazapine.

4. Maléate de S-mirtazapine, méthanesulfonate de S-mirtazapine, hydrate de fumarate de S-mirtazapine ou monohydrate de bromure de S-mirtazapine.
